# FASCICULE DE BREVET EUROPEEN

(11) **EP 0 764 437 B1**
(45) Date de publication et mention de la délivrance du brevet: **08.04.1998**
(21) Numéro de dépôt: 96401831.1
(22) Date de dépôt: 27.08.1996
(51) Int. Cl.: A61K 7/06

(54) **Composition aqueuse pour le maintien et/ou la fixation des cheveux comprenant un oligomère acrylique filmogène, soluble ou dispersible dans les milieux aqueux et utilisations**
Wässrige Zubereitung zur Plege und/oder Fixierung von Haaren enthaltend ein im wässrigen Milieu lösliches oder dispergierbares filmbildendes Acryloligomer sowie deren Verwendungen
Aqueous composition for the conditioning and/or fixation of hairs, containing a filmogen acrylic oligomer, soluble or dispersable in aqueous environments and its uses

(30) Priorité: 21.09.1995 FR 9511110
(43) Date de publication de la demande: 26.03.1997
(73) Titulaire: L'OREAL, 75008 Paris (FR)
(72) Inventeur: Samain, Henri, 91570 Bievres (FR); Mougin, Nathalie, 75011 Paris (FR)
(74) Mandataire: Miszputen, Laurent

(56) Documents cités:
- EP-A- 0 379 082
- EP-A- 0 590 604
- DE-A- 4 314 305
- FR-A- 2 351 135
- CHEMICAL ABSTRACTS, vol. 89, no. 14, 2 Octobre 1978 Columbus, Ohio, US; abstract no. 117546m, page 517; colonne 2; XP002000482 & JP-A-53 012 429 (ROHM AND HAAS CO.)

## Description

La présente invention a pour objet des compositions aqueuses pour la mise en forme et/ou le maintien de la chevelure, contenant dans un milieu aqueux cosmétiquement acceptable au moins un oligomère acrylique filmogène, soluble dans ledit milieu ainsi que ses utilisations.

Les polymères filmogènes solubles dans les milieux aqueux et hydroalcooliques tels que la polyvinylpyrrolidone, les copolymères de vinylpyrrolidone/acétate de vinyle, les copolymères d'acétate de vinyle/acide crotonique, les résines acryliques anioniques ou amphotères sont couramment utilisés dans les produits pour la mise en forme et/ou le maintien de la coiffure.

Les produits capillaires pour la mise en forme et/ou le maintien de la coiffure les plus répandus sur le marché de la cosmétique sont des compositions à pulvériser essentiellement constituées d'une solution le plus souvent hydroalcoolique et d'un polymère filmogène soluble dans l'eau et dans l'alcool tels que ceux cités ci-dessus, en mélange avec divers adjuvants cosmétiques. Cette solution est généralement conditionnée soit dans un récipient aérosol approprié mis sous pression à l'aide d'un gaz propulseur, soit dans un flacon pompe.

Depuis un certain nombre d'années, un intérêt tout particulier s'est manifesté pour les laques aérosols ou flacons pompes à fortes concentrations d'eau "à haut extrait sec" de polymère filmogène.

Dans toute la description, on entendra par aérosol ou flacon pompe à 〈〈haut extrait sec〉〉 en produit laquant, toute formulation aqueuse conditioneé sous l'une de ces formes contenant plus de 5% en poids en matière sèche de produit laquant par rapport au poids total de ladite formulation.

D'une part, on cherche à diminuer les concentrations en composés volatils à la pression atmosphérique dits COV (Composé Organique Volatile) présents dans les compositions à pulvériser sous forme d'aérosol ou de flacon pompe. En effet, l'emploi d'alcool, seul ou en mélange avec une faible quantité d'eau ainsi que l'utilisation de gaz propulseurs peut présenter certains inconvénients tels que l'augmentation de l'inflammabilité ou la nuisance vis-à-vis de l'environnement.

Les COV sont principalement les gaz propulseurs tels que les hydrocarbures ou le diméthyléther (DME) et les solvants tels que l'éthanol.

D'autre part, on cherche à réduire les temps de séchage du produit pulvérisé et à augmenter son pouvoir laquant après pulvérisation sur les cheveux. L'utilisation des polymères filmogènes hydrosolubles dans les aérosols ou flacons pompes à fortes concentrations d'eau, en particulier dans les systèmes pulvérisateurs eau/éthanol/diméthyléther ayant un taux de COV maximal de 55 %, nécessite des concentrations plus élevées en extrait sec de polymère par rapport à celles utilisées dans les systèmes pulvérisateurs organiques (100 % COV) pour obtenir des pouvoirs de fixation et des temps de séchage satisfaisants. En effet, l'augmentation de la concentration en eau dans les conditionnements aérosols ou flacons pompes conduit à une forte diminution du pouvoir laquant ainsi qu'à des temps de séchage beaucoup plus longs.

Les polymères filmogènes habituellement utilisés dans ce type de compositions ont des poids moléculaires mesurés en chromatographie d'exclusion stérique en général supérieurs à 50.000 et de préférence supérieurs à 100.000. L'augmentation de leur concentration dans des laques aérosols ou flacons pompes à fortes teneurs en eau, conduit à des viscosités trop élevées de sorte qu'on ne puisse plus obtenir une pulvérisation satisfaisante du produit à la sortie de l'aérosol ou du flacon pompe.

Pour remédier à ces problèmes de viscosité du 〈〈jus〉〉 dans le dispositif de pulvérisation, une solution consisterait à utiliser des oligomères filmogènes solubles ou dispersibles dans le milieu de la composition à pulvériser, apportant une faible viscosité. Or, la plupart des oligomères, de poids moléculaire inférieur à 50.000, utilisés dans les produits pour le maintien des cheveux présentent des propriétés mécaniques insuffisantes pour obtenir un pouvoir laquant satisfaisant, même à des concentrations élevées. Lorsque les oligomères, de poids moléculaire inférieur à 50.000, sont utilisés sous forme de latex (dispersion de particules), ils ont tendance à produire à la sortie du pulvérisateur un spray ou une laque d'aspect blanchâtre peu désirable sur le plan cosmétique et à être difficilement éliminables au shampooing.

On connaît dans la demande de brevet DE 43 14 305 des laques aérosols ou sprays pour la fixation des cheveux à base de copolymères acryliques de température de transition vitreuse Tg variant de 50 à 130°C, constitués (a) de méthacrylate de tertio-butyle ou d'acrylate de tertio-butyle ; (b) de monomère(s) à insaturation éthylénique comprenant une fonction carboxylique (acide (méth)acrylique) et (c) éventuellement d'un ou plusieurs monomères conduisant à un homopolymère ayant une Tg inférieur à 30°C.

On connaît également dans la demande de brevet EP-A-590 604 des laques aérosols ou sprays pour la fixation des cheveux à base de dispersions aqueuses de particules de copolymère acrylique, constitué (a) de méthacrylate d'alkyle en C₁-C₅ ; (b) d'acrylate d'alkyle en C₁-C₅ ; (c) de monomère(s) à insaturation éthylénique comprenant une fonction carboxylique (acide (méth)acrylique) ayant un poids moléculaire moyen de 10 000 à 50 000 et une température de transition vitreuse Tg variant de 10 à 50°C. Ce type de latex acryliques permet de réaliser des formulations à des taux de COV plus faibles qui sont stables aux variations de température pendant le transport ou le stockage.

La demanderesse a découvert de manière surprenante qu'en utilisant certains oligomères acryliques filmogènes, de poids moléculaire inférieur ou égal à 50.000 et de température de transition vitreuse Tg allant de 0 à 45° C, solubles dans les milieux aqueux, on pouvait réaliser des laques aérosols ou des flacons pompes à haut extrait sec en agent laquant, ayant une bonne diffusion lors de l'application, un bon pouvoir laquant, une bonne vitesse de séchage et de bonnes propriétés cosmétiques au niveau notamment du toucher et du démêlage. Ces oligomères particuliers que l'on définira plus loin, sont tout particulièrement adaptés aux compositions capillaires aérosols à 〈〈haut extrait sec〉〉 du type eau/éthanol/DME ayant un taux de COV maximal de 55 %. Ils sont de plus facilement éliminables au lavage et produisent à la sortie du pulvérisateur un spray, une laque ou une mousse dont l'aspect est satisfaisant sur le plan cosmétique.

Les compositions conformes à l'invention sont caractérisées par le fait qu'elles contiennent dans un milieu aqueux cosmétiquement acceptable au moins un oligomère acrylique filmogène, soluble dans ledit milieu, ayant un poids moléculaire mesuré par chromatographie d'exclusion stérique, inférieur ou égal à 50.000 et de température de transition vitreuse Tg allant de 0 à 45° C ; susceptible d'être obtenu par polymérisation d'un mélange de monomères comprenant :
a) de 20 à 45% en poids d'un monomère (A) choisi dans le groupe constitué par le méthacrylate de tertio-butyle, l'acrylate de tertio-butyle, le méthacrylate d'isobutyle et leurs mélanges ;
b) de 5 à 25 % en poids d'un monomère ou d'un mélange de monomères (B) à insaturation éthylénique comportant au moins une fonction acide carboxylique ;
c) au moins l'acrylate d'isobutyle (c) dans une quantité suffisante permettant d'obtenir un oligomère ayant une température Tg allant de 0 à 45° C ; les pourcentages en poids étant calculés par rapport à la quantité totale des monomères utilisés.

Les monomères (B) utilisés pour préparer les oligomères selon l'invention sont choisis par exemple dans le groupe constitué par : les monoacides carboxyliques à insaturation éthylènique tels que l'acide acrylique, l'acide méthacrylique et l'acide crotonique ; les diacides carboxyliques à insaturation éthylènique tels que l'acide maléique, l'acide fumarique, l'acide itaconique et leurs dérivés mono-esters ou mono-amides d'un groupe alkyle en C₁-C₄ ; l'acide allyloxyacétique. On utilise plus particulièrement l'acide acrylique, seul ou en mélange avec l'acide méthacrylique. Les monomères (B) sont utilisés, de préférence, à des concentrations allant de 8 à 15% en poids par rapport à la quantité totale des monomères utilisés.

Selon un mode particulier de l'invention, on utilise pour la préparation de l'oligomère un ou plusieurs monomères (D) à insaturation éthylénique complémentaires, copolymérisables avec les monomères (A), (B) et (C) et choisis de telle sorte que la température Tg de l'oligomère final soit de 0 à 45° C. Leur concentration varie de préférence de 0 à 10% en poids par rapport à la quantité totale des monomères utilisés.

Le ou les monomères (D) peuvent être choisis, par exemple, dans le groupe constitué par l'acétate de vinyle, le propionate de vinyle, le N-vinylpyrrolidone, le (méth)acrylate d'hydroxyéthyle, le (méth)acrylate de 2-hydroxypropyle, le (méth)acrylate de méthoxyéthyle, le (méth)acrylate d'éthoxyéthyle, le N,N-diméthylacrylamide ou leurs mélanges.

Les oligomères solubles dans le milieu aqueux des compositions de l'invention ont de préférence un poids moléculaire mesuré en chromatographie d'exclusion stérique allant de 500 à 45.000. Leur température Tg de transition vitreuse varie de 0 à + 45° C et plus particulièrement de +10 à + 35° C.

Les oligomères acryliques filmogènes selon l'invention peuvent être préparés de façon classique par polymérisation ou copolymérisation radicalaire en solution.

Ils sont préparés de préférence en solution dans un solvant organique à partir du ou des monomères en mélange dans le solvant en présence d'un initiateur de radicaux libres. Ils peuvent être ensuite purifés par précipitation dans un solvant tel que l'éther de pétrole.

On peut également opérer en semi-continu en utilisant un pied de cuve ne contenant que la partie solvant, une faible partie du mélange de monomères et une partie de l'amorceur. On chauffe ensuite à la température de réaction et on effectue une double coulée simultanée du reste du mélange des monomères et du reste de l'amorceur dissous dans une quantité de solvant.

Les oligomères acryliques de l'invention peuvent être ensuite partiellement ou totalement neutralisés par un composé monobasique non-volatil telle qu'une base minérale comme la soude ou la potasse, ou un aminoalcool par exemple pris dans le groupe constitué par l'amino-2 méthyl-2 propanol-1 (AMP), la triéthanolamine, la triisopropanolamine (TIPA), la monoéthanolamine, la tri[(2-hydroxy) 1-propyl]amine, l'amino-2 méthyl-2 propanediol-1,3 (AMPD), l'amino-2 hydroxyméthyl-2 propanediol-1,3.

Le milieu aqueux cosmétiquement acceptable de l'invention, est constitué de préférence par de l'eau ou un mélange d'eau et d'au moins un solvant cosmétiquement acceptable et compatible avec l'oligomère acrylique filmogène de l'invention tel qu'un monoalcool, un polyalcool, un éther de glycol, l'acétone ou un ester, seul ou sous forme de mélange. Il est plus particulièrement constitué d'eau ou d'eau et d'un alcool inférieur en C₁-C₄ comme l'éthanol ou l'isopropanol.

La concentration en solvant organique dans la composition de l'invention est de préférence comprise entre 15 et 35% en poids et plus particulièrement entre 20 et 30 % en poids par rapport au poids total de la composition.

Lorsque la composition selon l'invention est conditionnée sous pression dans un dispositif aérosol en vue d'obtenir une laque, elle comprend au moins un agent propulseur qui peut être choisi parmi les hydrocarbures volatils tels que le n-butane, le propane, l'isobutane, le pentane, les hydrocarbures chlorés et/ou fluorés et leurs mélanges ; le gaz carbonique, le protoxyde d'azote, le diméthyléther, l'azote ou l'air comprimé. La concentration du gaz propulseur dans le dispositif aérosol dépend de la nature du propulseur choisi.

Le diméthyléther est particulièrement préféré. Il est utilisé dans les laques aérosols de l'invention comme gaz propulseur dans des concentrations allant de préférence de 30 à 45% en poids par rapport au poids total de la composition.

La concentration en composé organique volatil (COV) dans une composition selon l'invention conditionnée sous forme d'aérosol ou de flacon pompe est de préférence inférieure ou égale à 55% en poids et plus particulièrement comprise entre 30 et 55% en poids par rapport au poids total de la formulation conditionée en aérosol ou en flacon pompe.

Le pH des compositions selon l'invention est généralement compris entre 2 et 9 et en particulier entre 3 et 8. Il peut être ajusté à la valeur choisie au moyen d'agents alcanisants ou acidifiants habituellement utilisés en cosmétique.

Les compositions selon l'invention peuvent contenir éventuellement en plus un agent plastifiant pour améliorer les propriétés mécaniques, les propriétés cosmétiques et l'adhésion sur les matières kératiniques de l'oligomère acrylique filmogène déposé après application et séchage. La présence d'un agent plastifiant n'est pas obligatoire pour ajuster le pouvoir laquant dans les formulations laques de l'invention contrairement aux formulations laques classiques.

Parmi les agents plastifiants pouvant être utilisés selon l'invention, on peut citer :
- les ®CARBITOLS de la Société UNION CARBIDE à savoir le ®CARBITOL ou diéthylène glycol éthyléther, le méthyl ®CARBITOL ou diéthylène glycol méthyléther, le butyl CARBITOL ou diéthylène glycol butyléther ou encore l'hexyl ®CARBITOL ou diéthylène glycol hexyléther,
- les ®CELLOSOLVES de la Société UNION CARBIDE à savoir le ®CELLOSOLVE ou éthylène glycol éthyléther, le butyl ®CELLOSOLVE ou éthylène glycol butyléther,
- l'hexyl ®CELLOSOLVE ou éthylène glycol hexyléther,
- les dérivés de propylène glycol et en particulier le propylène glycol phényléther, le propylène glycol diacétate, le dipropylène glycol butyléther, le tripropylène glycol butyléther, ainsi que les ®DOWANOLS de la Société DOW CHEMICAL à savoir le ®DOWANOL PM ou propylène glycolméthyléther, le ®DOWANOL DPM ou dipropylène glycol méthyléther et le ®DOWANOL TPM ou tripropylène glycol méthyléther.

On peut encore citer :
- le diéthylène glycol méthyléther ou ®DOWANOL DM de la Société DOW CHEMICAL,
- l'huile de ricin oxyéthylénée à 40 moles d'oxyde d'éthylène telle que celle vendue par la Société RHÔNE POULENC sous la dénomination de "®MULGOFEN LE-719",
- l'alcool benzylique,
- le citrate de triéthyle vendu par la Société PFIZER sous la dénomination de "®CITROFLEX-2",
- le 1,3-butylène glycol,
- les phtalates et adipates de diéthyle, de dibutyle et de diisopropyle,
- les tartrates de diéthyle et de dibutyle,
- les phosphates de diéthyle, de dibutyle et de diéthyl-2 hexyle, et
- les esters de glycérol tels que le diacétate de glycérol (diacétine) et le triacétate de glycérol (triacétine).

Les agents plastifiants sont choisis plus particulièrement parmi ceux qui sont hydrophiles ou solubles dans l'eau.

L'agent plastifiant est présent en une proportion allant, préférentiellement, de 0 à 20% en poids par rapport au poids de l'oligomère filmogène. Cette proportion varie selon l'application envisagée.

Les compositions selon l'invention telles que définies ci-dessus peuvent être utilisées comme produit coiffant pour la mise en forme et/ou le maintien de la coiffure choisi dans le groupe constitué par laques aérosol, les flacons pompes pour fixer les cheveux, les mousses aérosols de coiffage.

Les compositions capillaires pour le maintien de la coiffure conformes à la présente invention contiennent, de préférence, l'oligomère acrylique filmogène dans des proportions allant de 3 à 20 % en poids en matière sèche par rapport au poids total de la composition.

Les compositions capillaires conformes à l'invention peuvent contenir en plus des additifs cosmétiques conventionnels tels que des conservateurs, des adoucissants, des séquestrants, des parfums, des colorants, des modificateurs de viscosité, des agents nacrants, des agents hydratants, des agents antipelliculaires, des agents anti-séborrhéiques, des filtres solaires, des agents de conditionnement pour les cheveux, des anti-oxydants, des protéines, des vitamines.

Un autre objet de l'invention consiste en un procédé non-thérapeutique pour mettre en forme et/ou maintenir les cheveux, caractérisé en ce qu'il consiste à appliquer directement sur les cheveux une composition telle que définie précédemment.

Les exemples qui suivent servent à illustrer l'invention sans toutefois présenter un caractère limitatif.

### EXEMPLES

### EXEMPLE 1 : Préparation d'un oligomère acrylique filmogène compatible dans un milieu laque aérosol eau/éthanol/DME à taux de COV maximal de 55%

### Composition de l'oligomère :

| | | |
|---|---|---|
| - acrylate de tertio-butyle | (A) | 40% en poids |
| - acide acrylique | (B) | 10% en poids |
| - acrylate d'isobutyle | (C) | 50% en poids |

### Mode opératoire :

Dans un réacteur cylindrique avec agitation centrale mécanique, thermomètre, réfrigérant et barbotage d'azote on introduit le mélange suivant :

| | |
|---|---|
| - acrylate d'isobutyle | 50 g |
| - acrylate de tertio-butyle | 40 g |
| - acide méthacrylique | 10 g |
| - azobisisobutylacrylonitrile (amorceur) | 2 g |
| - éthanol | 200 g |

On porte sous agitation et barbotage d'azote au reflux de l'éthanol (78 °C). On laisse réagir 12 heures dans ces conditions. On revient à température ambiante. On purifie ensuite le polymère par précipitation de la solution alcoolique dans 5 l d'éther de pétrole. On sèche ensuite le précipité jusqu'à obtention d'un poids constant.

Le rendement obtenu après séchage est de 90%. L'indice d'acide obtenu est 81,5. Le poids moléculaire en sommet de pic mesuré en chromatographie d'exclusion stérique est de 36800 (élution dans le tétrahydrofurane par rapport à des étalons polystyrène). La température Tg mesurée par DSC (Calorimétrie Différentielle) est de 27 °C.

On réalise une solution alcoolique concentré d'oligomère neutralisé à 100% par l'amino-2 méthyl-2 propanol-1 (AMP) à partir du mélange suivant :

| | |
|---|---|
| - Oligomère | 100 g |
| - AMP | 12,44 g |
| - Ethanol | 112,44 g |

On agite pendant 24 heures à température ambiante. On obtient ainsi une solution à 50% en poids en matière sèche d'oligomère.

### EXEMPLE 2 : Laque aérosol eau/éthanol/DME à 45% de COV pour la fixation des cheveux contenant l'oligomère acrylique de l'exemple 1

### COMPOSITION AVANT CONDITIONNEMENT :

| | |
|---|---|
| - Solution alcoolique à 50% en poids d'oligomère de l'exemple 2 | 14 g |
| - Eau permutée | 48 g |
| - Ethanol | 8 g |

### LAQUE AEROSOL A 45% EN COV :

| | |
|---|---|
| - Composition ci-dessus | 70 g |
| - Diméthyléther | 30 g |

Cette laque contient 7% en poids de matière sèche

Après application sur les cheveux, on obtient un excellent pouvoir laquant, un temps de séchage rapide, de bonnes propriétés cosmétiques notamment au niveau du toucher et du démêlage et une bonne élimination du dépôt au shampooing.

### EXEMPLE 3 : Laque aérosol eau/éthanol/DME à 45% de COV pour la fixation des cheveux contenant l'oligomère acrylique de l'exemple 1

### COMPOSITION AVANT CONDITIONNEMENT :

| | |
|---|---|
| - Solution alcoolique à 50% en poids d'oligomère de l'exemple 1 | 21,0 g |
| - Eau permutée | 44,5 g |
| - Ethanol | 4,5 g |

### LAQUE AEROSOL A 45% EN COV :

| | |
|---|---|
| - Composition ci-dessus | 70 g |
| - Diméthyléther | 30 g |

Cette laque contient 7% en poids de matière sèche

Après application sur les cheveux, on obtient un excellent pouvoir laquant, un temps de séchage rapide, de bonnes propriétés cosmétiques notamment au niveau du toucher et du démêlage et une bonne élimination du dépôt au shampooing.

## Revendications

1. Composition pour la mise en forme et/ou le maintien de la chevelure, caractérisée par le fait qu'elle contient dans un milieu aqueux cosmétiquement acceptable au moins un oligomère acrylique filmogène soluble dans ledit milieu ayant un poids moléculaire mesuré par chromatographie d'exclusion stérique, inférieur ou égal à 50.000 et de température de transition vitreuse Tg allant de 0 à 45° C ; ledit oligomère étant susceptible d'être obtenu par polymérisation radicalaire en solution d'un mélange de monomères comprenant au moins :
a) de 20 à 45% en poids d'un monomère (A) choisi dans le groupe constitué par le méthacrylate de tertio-butyle, l'acrylate de tertio-butyle, le méthacrylate d'isobutyle et leurs mélanges ;
b) de 5 à 25 % en poids d'un monomère ou d'un mélange de monomères (B) à insaturation éthylénique comportant au moins une fonction acide carboxylique ;
c) de l'acrylate d'isobutyle (C) dans une quantité suffisante permettant d'obtenir un oligomère ayant une température Tg allant de 0 à 45°C ; les pourcentages en poids étant calculés par rapport à la quantité totale des monomères utilisés.

2. Composition selon la revendication 1, caractérisée par le fait que le ou les monomères (B) sont utilisés à des concentrations allant de 8 à 15 % en poids par rapport à la quantité totale des monomères utilisés.

3. Composition selon la revendication 1 ou 2, caractérisée par le fait que le ou les monomères (B) utilisés pour préparer les oligomères selon l'invention sont choisis dans le groupe constitué par : les monoacides carboxyliques à insaturation éthylènique ; les diacides carboxyliques à insaturation éthylènique et leurs dérivés mono-esters ou mono-amides d'un groupe alkyle en C₁-C₄ ; l'acide allyloxyacétique.

4. Composition selon l'une quelconque des revendications 1 à 3, caractérisée par le fait que le ou les monomères (B) utilisés pour préparer les oligomères selon l'invention sont choisis dans le groupe constitué par l'acide acrylique, l'acide méthacrylique ou leur mélange.

5. Composition selon l'une quelconque des revendications 1 à 4, caractérisée par le fait que l'oligomère comprend en plus un ou plusieurs monomères (D) à insaturation éthylénique complémentaires, copolymérisables avec les monomères (A), (B) et (C) et choisis de telle sorte que la température Tg de l'oligomère final soit de 0 à 45° C.

6. Composition selon la revendications 5, caractérisée par le fait que le ou les monomères (D) sont utilisés à des concentrations allant de 0 à 10 % en poids par rapport à la quantité totale des monomères utilisés.

7. Composition selon la revendications 5 ou 6, caractérisée par le fait que le ou les monomères (D) sont choisis dans le groupe constitué par l'acétate de vinyle, le propionate de vinyle, le N-vinylpyrrolidone, le (méth)acrylate d'hydroxyéthyle, le (méth)acrylate de 2-hydroxypropyle, le (méth)acrylate de méthoxyéthyle, le (méth)acrylate d'éthoxyéthyle, le N,N-diméthylacrylamide ou leurs mélanges.

8. Composition selon l'une quelconque des revendications 1 à 7, caractérisée par le fait que l'oligomère filmogène a un poids moléculaire mesuré en chromatographie d'exclusion stérique allant de 500 à 45.000.

9. Composition selon l'une quelconque des revendications 1 à 8, caractérisée par le fait que l'oligomère filmogène a une température de transition vitreuse Tg allant de +10 °C à + 35 °C.

10. Composition selon l'une quelconque des revendications 1 à 9, caractérisée par le fait que le milieu aqueux cosmétiquement acceptable est constitué par de l'eau ou un mélange d'eau et d'au moins un solvant cosmétiquement acceptable et compatible avec l'oligomère filmogène.

11. Composition selon la revendication 10, caractérisée par le fait que le solvant est choisi dans le groupe constitué par des monoalcools, des polyalcools, des éthers de glycol ou des esters, l'acétone, seuls ou en mélange.

12. Composition selon l'une quelconque des revendications 1 à 11, caractérisée par le fait que le milieu aqueux cosmétiquement acceptable est constitué d'eau ou d'eau et d'un alcool inférieur en C₁-C₄.

13. Composition selon l'une quelconque des revendications 10 à 12, caractérisée par le fait que la concentration en solvant organique est comprise entre 15 et 35 % en poids par rapport au poids total de la composition.

14. Composition selon l'une quelconque des revendications 1 à 13, caractérisée par le fait qu'elle est conditionnée dans un dispositif aérosol sous pression ou un flacon pompe.

15. Composition selon la revendication 14, caractérisée par le fait qu'elle est conditionnée dans un dispositif aérosol sous pression en présence d'un agent propulseur.

16. Composition selon la revendication 15, selon laquelle l'agent propulseur est choisi dans le groupe constitué par les hydrocarbures volatils, les hydrocarbures chlorés et/ou fluorés et leurs mélanges ; le gaz carbonique, le protoxyde d'azote, le diméthyléther, l'azote ou l'air comprimé.

17. Composition selon la revendication 15 ou 16, selon laquelle l'agent propulseur est le diméthyléther.

18. Composition selon la revendication 17, selon laquelle la concentration en diméthyléther est comprise entre 30 et 45% en poids par rapport au poids total de la composition.

19. Composition selon la revendication 14, selon laquelle la concentration en composé organique volatil (COV) est inférieure ou égale à 55% en poids par rapport au poids total de la composition conditionnée en aérosol ou en flacon pompe.

20. Composition selon l'une quelconque des revendications 1 à 19, caractérisée par le fait qu'elle contient en plus un agent plastifiant.

21. Composition selon l'une quelconque des revendications 1 à 20, caractérisée par le fait qu'elle contient l'oligomère filmogène dans des proportions allant de 3 à 20 % en poids en matière sèche par rapport au poids total de la composition.

22. Composition selon l'une quelconque des revendications 1 à 21, caractérisée par le fait qu'il s'agit d'un produit de coiffage choisi dans le groupe constitué par les laques aérosols, les flacons pompes pour fixer les cheveux et les mousses aérosols de coiffage.

23. Procédé non-thérapeutique de mise en forme et/ou de fixation de la chevelure caractérisé en ce qu'il consiste à appliquer directement sur les cheveux une composition cosmétique telle que définie selon l'une quelconque des revendications 1 à 22.

## Claims

1. Composition for hair shaping and/or form retention of hair, characterized in that it contains, in a cosmetically acceptable aqueous medium, at least one film-forming acrylic oligomer, which is soluble in the said medium, having a molecular weight, measured by steric exclusion chromatography, of less than or equal to 50,000 and with a glass transition temperature Tg ranging from 0 to 45°C, the said oligomer being capable of being obtained by solution radical polymerization of a mixture of monomers comprising at least:
a) from 20 to 45 weight % of a monomer (A) chosen from the group composed of tert-butyl methacrylate, tert-butyl acrylate, isobutyl methacrylate and their mixtures;
b) from 5 to 25 weight % of a monomer or of a mixture of monomers (B) with ethylenic unsaturation containing at least one carboxylic acid functional group;
c) isobutyl acrylate (C) in an amount which is sufficient to enable an oligomer having a Tg temperature ranging from 0 to 45°C to be obtained, the percentages by weight being calculated with respect to the total amount of the monomers used.

2. Composition according to Claim 1, characterized in that the monomer or monomers (B) are used at concentrations ranging from 8 to 15 weight % with respect to the total amount of the monomers used.

3. Composition according to Claim 1 or 2, characterized in that the monomer or monomers (B) used to prepare the oligomers according to the invention are chosen from the group composed of: monocarboxylic acids with ethylenic unsaturation; dicarboxylic acids with ethylenic unsaturation and their monoester or monoamide derivatives with a C₁-C₄ alkyl group; or allyloxyacetic acid.

4. Composition according to any one of Claims 1 to 3, characterized in that the monomer or monomers (B) used to prepare the oligomers according to the invention are chosen from the group composed of acrylic acid, methacrylic acid or their mixture.

5. Composition according to any one of Claims 1 to 4, characterized in that the oligomer additionally comprises one or a number of complementary monomers (D) with ethylenic unsaturation which are copolymerizable with the monomers (A), (B) and (C) and which are chosen so that the Tg temperature of the final oligomer is from 0 to 45°C.

6. Composition according to Claim 5, characterized in that the monomer or monomers (D) are used at concentrations ranging from 0 to 10 weight % with respect to the total amount of the monomers used.

7. Composition according to Claim 5 or 6, characterized in that the monomer or monomers (D) are chosen from the group composed of vinyl acetate, vinyl propionate, N-vinylpyrrolidone, hydroxyethyl (meth)acrylate, 2-hydroxypropyl (meth)acrylate, methoxyethyl (meth)acrylate, ethoxyethyl (meth)acrylate, N,N-dimethylacrylamide or their mixtures.

8. Composition according to any one of Claims 1 to 7, characterized in that the film-forming oligomer has a molecular weight, measured by steric exclusion chromatography, ranging from 500 to 45,000.

9. Composition according to any one of Claims 1 to 8, characterized in that the film-forming oligomer has a glass transition temperature Tg ranging from +10°C to +35°C.

10. Composition according to any one of Claims 1 to 9, characterized in that the cosmetically acceptable aqueous medium is composed of water or a mixture of water and of at least one cosmetically acceptable solvent which is compatible with the film-forming oligomer.

11. Composition according to Claim 10, characterized in that the solvent is chosen from the group composed of monoalcohols, polyalcohols, glycol ethers, or esters, acetone, alone or as a mixture.

12. Composition according to any one of Claims 1 to 11, characterized in that the cosmetically acceptable aqueous medium is composed of water or of water and of a lower C₁-C₄ alcohol.

13. Composition according to any one of Claims 10 to 12, characterized in that the concentration of organic solvent is between 15 and 35 weight % with respect to the total weight of the composition.

14. Composition according to any one of Claims 1 to 13, characterized in that it is packaged in an aerosol device under pressure or a pump-action spray.

15. Composition according to Claim 14, characterized in that it is packaged in an aerosol device under pressure in the presence of a propellent agent.

16. Composition according to Claim 15, according to which the propellent agent is chosen from the group composed of volatile hydrocarbons or chlorinated and/or fluorinated hydrocarbons and their mixtures, carbon dioxide gas, nitrous oxide, dimethyl ether, nitrogen or compressed air.

17. Composition according to Claim 15 or 16, according to which the propellent agent is dimethyl ether.

18. Composition according to Claim 17, according to which the concentration of dimethyl ether is between 30 and 45 weight % with respect to the total weight of the composition.

19. Composition according to Claim 14, according to which the concentration of volatile organic compound (VOC) is less than or equal to 55 weight % with respect to the total weight of the composition packaged as an aerosol or as a pump-action spray.

20. Composition according to any one of Claims 1 to 19, characterized in that it additionally contains a plasticizing agent.

21. Composition according to any one of Claims 1 to 20, characterized in that it contains the film-forming oligomer in proportions ranging from 3 to 20 weight %, on a dry basis, with respect to the total weight of the composition.

22. Composition according to any one of Claims 1 to 21, characterized in that it concerns a styling product chosen from the group composed of aerosol lacquers, pump-action sprays for fixing the hair and styling aerosol foams.

23. Non-therapeutic process for hair shaping and/or for fixing hair, characterized in that it comprises the direct application to hair of a cosmetic composition as defined according to any one of Claims 1 to 22.

## Patentansprüche

1. Zusammensetzung für die Formung/Gestaltung und den Halt der Haare, dadurch gekennzeichnet, daß sie in einem kosmetisch akzeptablen wäßrigen Medium mindestens ein in diesem Medium lösliches filmbildendes Acryloligomer enthält, das ein durch sterische Ausschlußchromatographie gemessenes Molekulargewicht von höchstens 50 000 und eine Glasübergangstemperatur Tg von 0 bis 45 °C aufweist, wobei das Oligomer durch radikalische Lösungspolymerisation eines Monomerengemischs hergestellt werden kann, das mindestens enthält
a) 20 bis 45 Gew.-% eines Monomers (A), das unter *tert*.-Butylmethacrylat, *tert*.-Butylacrylat, Isobutylmethacrylat und deren Gemischen ausgewählt ist,
b) 5 bis 25 Gew.-% eines ethylenisch ungesättigten Monomers oder eines Gemischs von ethylenisch ungesättigten Monomeren (B), das/die mindestens eine Carboxygruppe aufweist/aufweisen,
c) Isobutylacrylat (C) in einer ausreichenden Menge, die es ermöglicht, ein Oligomer mit einer Temperatur Tg von 0 bis 45 °C herzustellen,
wobei die Gewichtsprozentangaben auf die Gesamtmenge der verwendeten Monomere bezogen sind.

2. Zusammensetzung nach Anspruch 1, dadurch gekennzeichnet, daß das oder die Monomeren (B) in einer Konzentration von 8 bis 15 Gew.-%, bezogen auf die Gesamtmenge der verwendeten Monomere, verwendet werden.

3. Zusammensetzung nach Anspruch 1 oder 2, dadurch gekennzeichnet, daß das oder die Monomere (B), die für die Herstellung der erfindungsgemäßen Oligomeren verwendet werden, unter ethylenisch ungesättigten Monocarbonsäuren, ethylenisch ungesättigten Dicarbonsäuren und deren Monoester- oder Monoamid-Derivaten mit einer C₁₋₄-Alkylgruppe und Allyloxyessigsäure ausgewählt sind.

4. Zusammensetzung nach einem der Ansprüche 1 bis 3, dadurch gekennzeichnet, daß das oder die Monomere (B), die für die Herstellung der erfindungsgemäßen Oligomere verwendet werden, unter Acrylsäure, Methacrylsäure und deren Gemischen ausgewählt sind.

5. Zusammensetzung nach einem der Ansprüche 1 bis 4, dadurch gekennzeichnet, daß das Oligomer zusätzlich ein oder mehrere ergänzende, ethylenisch ungesättigte Monomere (D) enthält, die mit den Monomeren (A), (B) und (C) copolymerisierbar sind und so ausgewählt sind, daß die Temperatur Tg des als Endprodukt erhaltenen Oligomers 0 bis 45 °C beträgt.

6. Zusammensetzung nach Anspruch 5, dadurch gekennzeichnet, daß das oder die Monomere (D) in Konzentrationen verwendet werden, die 0 bis 10 Gew.-%, bezogen auf die gesamte Menge der verwendeten Monomeren, betragen.

7. Zusammensetzung nach Anspruch 5 oder 6, dadurch gekennzeichnet, daß das oder die Monomeren (D) unter Vinylacetat, Vinylpropionat, N-Vinylpyrrolidon, Hydroxyethyl(meth)acrylat, 2-Hydroxypropyl(meth)acrylat, Methoxyethyl(meth)acrylat, Ethoxyethyl(meth)acrylat, N,N-Dimethylacrylamid und deren Gemischen ausgewählt sind.

8. Zusammensetzung nach einem der Ansprüche 1 bis 7, dadurch gekennzeichnet, daß das filmbildende Oligomer ein durch sterische Ausschlußchromatographie gemessenes Molekulargewicht von 500 bis 45 000 aufweist.

9. Zusammensetzung nach einem der Ansprüche 1 bis 8, dadurch gekennzeichnet, daß das filmbildende Oligomer eine Glasübergangstemperatur Tg von +10 °C bis +35 °C aufweist.

10. Zusammensetzung nach einem der Ansprüche 1 bis 9, dadurch gekennzeichnet, daß das kosmetisch akzeptable wäßrige Medium aus Wasser oder einem Gemisch aus Wasser und mindestens einem kosmetisch akzeptablen und mit dem filmbildenden Polymer kompatiblen Lösungsmittel besteht.

11. Zusammensetzung nach Anspruch 10, dadurch gekennzeichnet, daß das Lösungsmittel unter einwertigen Alkoholen, Polyalkoholen, Glykolethern und Estern, Aceton, allein oder im Gemisch, ausgewählt ist.

12. Zusammensetzung nach einem der Ansprüche 1 bis 11, dadurch gekennzeichnet, daß das kosmetisch akzeptable wäßrige Medium aus Wasser oder Wasser und einem niederen C₁₋₄-Alkohol besteht.

13. Zusammensetzung nach einem der Ansprüche 10 bis 12, dadurch gekennzeichnet, daß die Konzentration an organischem Lösungsmittel 15 bis 35 Gew.-%, bezogen auf das Gesamtgewicht der Zusammensetzung, beträgt.

14. Zusammensetzung nach einem der Ansprüche 1 bis 13, dadurch gekennzeichnet, daß sie in einer unter Druck stehenden Aerosol-Vorrichtung oder einer Pumpflasche verpackt ist.

15. Zusammensetzung nach Anspruch 14, dadurch gekennzeichnet, daß sie in einer unter Druck stehenden Aerosol-Vorrichtung in Gegenwart eines Treibmittels konfektioniert ist.

16. Zusammensetzung nach Anspruch 15, wobei das Treibmittel unter flüchtigen Kohlenwasserstoffen, chlorierten und/oder fluorierten Kohlenwasserstoffen und deren Gemischen, Kohlendioxid, Distickstoffmonoxid, Dimethylether, Stickstoff, komprimierter Luft ausgewählt ist.

17. Zusammensetzung nach Anspruch 15 oder 16, wobei es sich bei dem Treibmittel um Dimethylether handelt.

18. Zusammensetzung nach Anspruch 17, wobei die Konzentration an Dimethylether 30 bis 45 Gew.-%, bezogen auf das Gesamtgewicht der Zusammensetzung, beträgt.

19. Zusammensetzung nach Anspruch 14, wobei die Konzentration an flüchtiger organischer Verbindung (= COV von "composé organique volatil") höchstens 55 Gew.-%, bezogen auf das Gesamtgewicht der als Aerosol oder in einer Pumpflasche konfektionierten Zusammensetzung, beträgt.

20. Zusammensetzung nach einem der Ansprüche 1 bis 19, dadurch gekennzeichnet, daß sie außerdem einen Weichmacher enthält.

21. Zusammensetzung nach einem der Ansprüche 1 bis 20, dadurch gekennzeichnet, daß sie das filmbildende Oligomer in einem Anteil von 3 bis 20 Gew.-% Trockensubstanz, bezogen auf das Gesamtgewicht der Zusammensetzung, enthält.

22. Zusammensetzung nach einem der Ansprüche 1 bis 21, dadurch gekennzeichnet, daß es sich um ein Frisierprodukt handelt, das unter Aerosollaeken, in Pumpflaschen konfektionierten Zusammensetzungen zur Festigung der Haare und Aerosolschäumen zum Frisieren ausgewählt ist.

23. Nicht-therapeutisches Verfahren zur Formung der Haare und/oder für den Halt der Haare, dadurch gekennzeichnet, daß es darin besteht, eine kosmetische Zusammensetzung nach einem der Ansprüche 1 bis 22 direkt auf die Haare aufzutragen.
